# EUROPEAN PATENT APPLICATION

(11) **EP 2 347 700 A1**
(43) Date of publication of application: **27.07.2011**
(21) Application number: 10815276.0
(22) Date of filing: 27.08.2010
(51) Int. Cl.: A61B 1/12, G02B 23/24

(54) **ENDOSCOPE CLEANING TOOL AND ENDOSCOPE CLEANING APPARATUS**

(30) Priority: 14.09.2009 JP 2009212246
(71) Applicant: OLYMPUS MEDICAL SYSTEMS CORP., Tokyo 151-0072 (JP)
(72) Inventor: SUZUKI, Eiri, Tokyo 151-0072 (JP); ONISHI, Hideto, Tokyo 151-0072 (JP); KAWASE, Takahiko, Tokyo 151-0072 (JP)
(74) Representative: Schicker, Silvia
(86) International application number: PCT/JP2010/064601
(87) International publication number: WO 2011/030679

(57) **Abstract**

An endoscope cleaning instrument and an endoscope cleaning apparatus are provided that have a simple configuration and that can be manufactured easily and at a low cost. An endoscope cleaning instrument of the invention cleans a distal end portion of an endoscope by ejecting a fluid fed from a fluid supply section towards the distal end portion. The endoscope cleaning instrument includes a connection portion that is attachable to and detachable from the fluid supply section; at least one tubular portion that has a tube shape including an inner circumferential portion and an outer circumferential portion and that can introduce the fluid supplied from the fluid supply section via the connection portion into an inner space enclosed by the inner circumferential portion, and that is provided so as to enclose an outer space that can accommodate the distal end portion by means of at least a part of the outer circumferential portion; and a plurality of through-holes provided in the tubular portion so as to connect the inner space and the outer space.

## Description

### Technical Field

The present invention relates to an endoscope cleaning instrument and an endoscope cleaning apparatus that clean an endoscope by ejecting a fluid towards the endoscope.

### Background Art

Endoscopes that are used in the medical field are subjected to a cleaning process after use. A distal end portion of an insertion portion of an endoscope has a concavo-convex shape. This is because an opening such as a treatment instrument insertion port or a fluid delivery port, and an observation section such as an optical window or an ultrasound transmitting/receiving section are provided at the distal end portion. Accordingly, in a cleaning process for the endoscope, it is preferable that priority is given to cleaning the distal end portion of the insertion portion that has the concavo-convex shape to which dirt is liable to adhere.

For example, Japanese Utility Model Laid-Open No. 55-116523 discloses an endoscope cleaning instrument in which an injection port through which a cleaning fluid is injected towards an inner side is provided in a cylindrical member into which an insertion portion of an endoscope can be inserted, and which cleans the distal end portion of an endoscope that is inserted inside the cylindrical member.

However, according to the configuration of the endoscope cleaning instrument disclosed in Japanese Utility Model Laid-Open No. 55-116523, the cylindrical members are doubly coupled and the cleaning fluid is injected towards the inner side. Therefore, a large number of members are required and the configuration is complicated.

The present invention has been achieved in view of the above-described points, and an object of the present invention is to provide an endoscope cleaning instrument and an endoscope cleaning apparatus that have a simple configuration and can be manufactured easily and at a low cost.

### Disclosure of Invention

### Means for Solving the Problem

An endoscope cleaning instrument according to the present invention cleans a distal end portion of an endoscope by ejecting a fluid fed from a fluid supply section towards the distal end portion, including: a connection portion that is attachable to and detachable from the fluid supply section; at least one tubular portion that has a tube shape including an inner circumferential portion and an outer circumferential portion and that can introduce the fluid that is supplied from the fluid supply section via the connection portion into an inner space enclosed by the inner circumferential portion, and that is provided so as to enclose an outer space capable of accommodating the distal end portion by means of at least a part of the outer circumferential portion; and a plurality of through-holes provided in the tubular portion so as to connect the inner space and the outer space.

An endoscope cleaning apparatus according to the present invention includes the endoscope cleaning instrument.

According to the present invention, an endoscope cleaning instrument and an endoscope cleaning apparatus can be provided that have a simple configuration and that can be manufactured easily and at a low cost.

### Brief Description of the Drawings

Fig. 1 is a view that illustrates the configuration of an endoscope cleaning instrument according to a first embodiment;
Fig. 2 is a side view of a tubular portion;
Fig. 3 is a cross-sectional view of the tubular portion;
Fig. 4 is a view that illustrates the manner of performing a cleaning process that uses the endoscope cleaning instrument of the first embodiment;
Fig. 5 is a view that illustrates a modification example of a fixing section;
Fig. 6 is an oblique perspective view of a tubular portion of an endoscope cleaning instrument according to a second embodiment;
Fig. 7 is a cross-sectional view of the tubular portion of the endoscope cleaning instrument according to the second embodiment;
Fig. 8 is an external view of an endoscope cleaning apparatus; and
Fig. 9 is a view that illustrates a state in which an endoscope and an endoscope cleaning instrument are arranged inside a cleaning tank prior to a cleaning process.

### Best Mode for Carrying Out the Invention

Hereunder, preferred embodiments of the present invention will be described with reference to the drawings. Note that, in each of the drawings referred to in the description below, scale sizes are differentiated for each component in order to show each component in a recognizable size in the drawings. The present invention is not limited only to the number, the shape, and the size ratio of the components illustrated in the drawing, or to the relative positional relationship between the components.

### (First Embodiment)

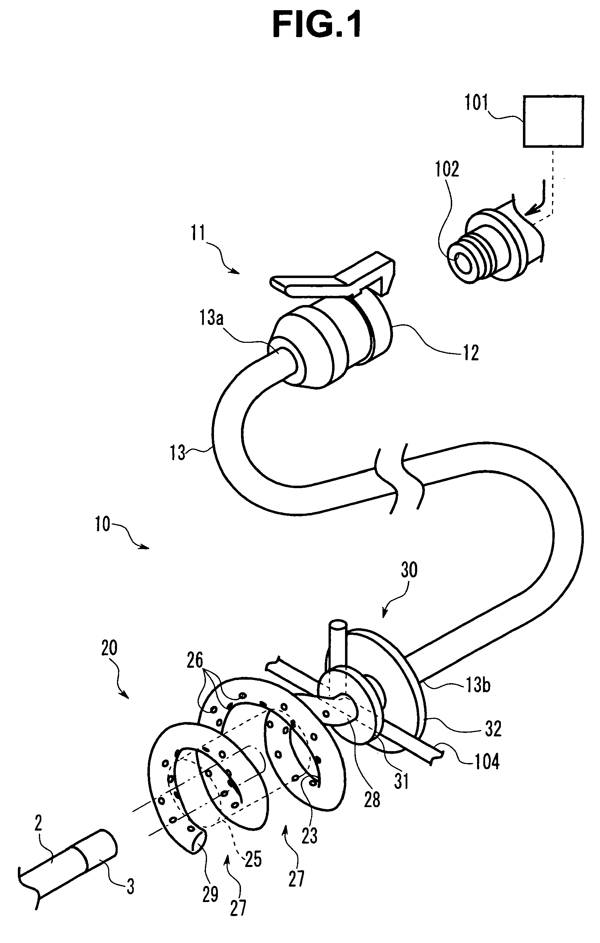

Hereunder, a first embodiment of the present invention will be described referring to Fig. 1 to Fig. 4. An endoscope cleaning instrument 10 of the present embodiment shown in Fig. 1 is an instrument for cleaning a distal end portion 3 of an insertion portion 2 of an endoscope by ejecting a fluid towards the distal end portion 3 from the area surrounding the distal end portion 3.

Note that the form of the endoscope is not particularly limited. For example, the endoscope may include a flexible insertion portion 2, or may be a so-called "rigid endoscope" that includes a rigid insertion portion 2. Further, the endoscope may include an optical observation section such as an observation window or illumination for performing optical observation in the insertion portion, or may include an ultrasound transmitting/receiving section for performing transmission and reception of ultrasound to conduct ultrasound observation in the insertion portion. The endoscope may also include a complex mechanism such as a forceps raising base at a distal end. Since such endoscopes are known, a detailed description of an endoscope is omitted herein.

The fluid that is ejected towards the distal end portion 3 of the insertion portion 2 of the endoscope to clean the distal end portion 3 includes at least one of a gas and a liquid. The fluid may be in the form of a gas-liquid fluid mixture in which a gas and a liquid are mixed, or may have a form in which a powdered solid is mixed in at least one of a gas and a liquid.

The form and type of the fluid are not particularly limited as long as the fluid can be used in a cleaning process of the distal end portion 3. As one example according to the present embodiment, the fluid is a liquid such as water, a cleaning chemical, or alcohol.

According to the present embodiment, the fluid is supplied from a fluid supply section 101. The fluid supply section 101 is not particularly limited as long as the fluid supply section 101 ejects a fluid at a predetermined pressure from a fluid supply port 102. For example, the fluid supply section 101 may be equipment such as a water supply that is provided in a facility, or may be an apparatus that discharges a fluid by means of the force of gravity or the action of a pump.

As one example according to the present embodiment, the fluid supply section 101 is arranged in an unshown endoscope cleaning apparatus, and supplies a fluid by means of an electrically-driven pump. The endoscope cleaning apparatus has a cleaning tank that is capable of accommodating an endoscope, and performs a cleaning process of an endoscope using at least a fluid inside the cleaning tank.

The endoscope cleaning instrument 10 includes a connection portion 11 and a tubular portion 20. The connection portion 11 is capable of connecting to a fluid supply port 102 of the fluid supply section 101, and has a configuration that causes fluid that is discharged from the fluid supply port 102 to be introduced into the tubular portion 20 that is described later.

Although the configuration of the connection portion 11 is not particularly limited, preferably the connection portion 11 is attachable to and detachable from the fluid supply port 102. If the connection portion 11 is attachable to and detachable from the fluid supply port 102 of the fluid supply section 101, the cleaning process of the endoscope cleaning instrument 10 itself and the cleaning process of the fluid supply section 101 can be performed with ease.

As one example according to the present embodiment, the connection portion 11 includes a connector 12 and a tubular connection tube portion 13. The connector 12 is attachable to and detachable from the fluid supply port 102 of the fluid supply section 101, and has a configuration that connects a proximal end 13a of the connection tube portion 13 and the fluid supply port 102 when attached to the fluid supply port 102.

The connection tube portion 13 is a flexible tubular member in which the proximal end 13a is connected to the connector 12 and a distal end 13b is connected to the tubular portion 20 that is described later. In this connection, the connection tube portion 13 may have a cylindrical configuration that is not flexible, and that simply connects the connector 12 and the tubular portion 20.

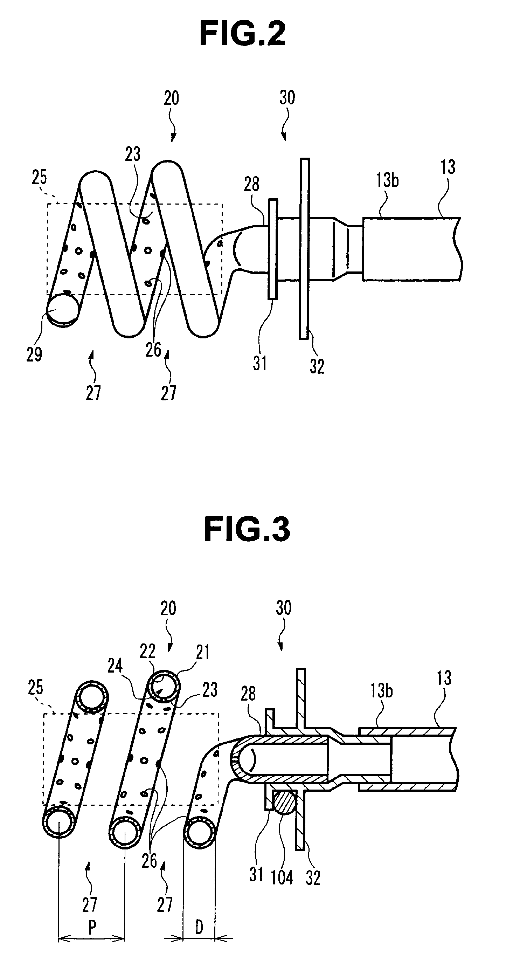

The tubular portion 20 has a configuration that ejects a fluid that is supplied from the fluid supply section 101 via the connection portion 11, towards the distal end portion 3 of the insertion portion 2 of the endoscope from the area surrounding the distal end portion 3. The tubular portion 20 has a tube shape in which a proximal end 28 is connected to the distal end 13b of the connection tube portion 13, and a distal end 29 is closed. However, a hole may also be provided in the distal end 29 to thereby easily decrease the amount of fluid remaining inside the tubular portion 20 after using the tubular portion 20. As shown in the cross-sectional view in Fig. 3, the tube-shaped tubular portion 20 includes an inner circumferential portion 22 and an outer circumferential portion 23 of a tube wall 21 constituting the tubular portion 20.

An inner space 24 enclosed by the inner circumferential portion 22 of the tubular portion 20 communicates with the connection tube portion 13. More specifically, when the connection portion 11 is attached to the fluid supply port 102 of the fluid supply section 101, the inner space 24 of the tubular portion 20 communicates with the fluid supply port 102 via the connection portion 11.

The tubular portion 20 is provided so that the circumference of an outer space 25 that can accommodate the distal end portion 3 of the insertion portion 2 of the endoscope is enclosed by at least a part of the outer circumferential portion 23. As illustrated by a wavy line in the drawings, the outer space 25 that can accommodate the distal end portion 3 is an approximately cylindrical space that has a predetermined cross-sectional area and a predetermined length into which the distal end portion 3 can be inserted.

A plurality of through-holes 26 that penetrate the tube wall 21 so as to connect the inner space 24 and the outer space 25 are provided in the tubular portion 20. In other words, through-holes 26 that extend as far as the inner circumferential portion 22 are provided in a region (region facing the outer space 25) enclosing the outer space 25 of the outer circumferential portion 23 of the tubular portion 20.

More specifically, the tubular portion 20 has a tube shape in which the tube wall 21 covers an inner space 24 into which fluid supplied from the fluid supply section 101 via the connection portion 11 can be introduced. The tubular portion 20 is configured such that it is possible to enclose the circumference of the distal end portion 3 of the insertion portion 2. Further, the tubular portion 20 has the plurality of through-holes 26 that penetrate through the tube wall 21 in a region facing the distal end portion 3.

Although the form of the tubular portion 20 is not particularly limited, as one example according to the present embodiment, the tubular portion 20 is constituted by a tubular member that has a circular cross-section that is formed in a helical shape so as to enclose the outer space 25. In other words, according to the present embodiment, a space enclosed by a helical tubular portion 20 is defined as the outer space 25 that can accommodate the distal end portion 3 of the insertion portion 2 of the endoscope.

As used herein, the term "helical" refers to a shape that winds so as to advance to one side along the axial direction of the outer space 25 as it goes towards the distal end 29 from the proximal end 28 while rotating around the circumference of the cylindrical outer space 25, and for example, this shape is also referred to as a spiral shape or a coil shape.

As shown in Fig. 3, the tubular portion 20 of the present embodiment is formed so that a helical winding pitch P is greater than a diameter D of a tubular member constituting the tubular portion. Accordingly, a clearance 27 in which the outer space 25 that is enclosed by the helical tubular portion 20, and the external portion of the endoscope cleaning instrument 10 communicate is provided in the tubular portion 20.

Although according to the present embodiment a configuration is adopted in which the tubular portion 20 is constituted by a tubular member with a circular cross-section, the tubular portion 20 may be constituted by a tubular member that has a cross-sectional shape such as an elliptical shape, a rectangular shape, or a polygonal shape. The material constituting the tubular portion 20 is not particularly limited as long as the material has resistance to a fluid that is used to clean the endoscope, such as a metal, ceramic, or synthetic resin material.

Further, although according to the present embodiment illustrated in the drawings the tubular portion 20 is formed so as to have an approximately circular shape when viewed from the axial direction of the outer space 25, the shape of the tubular portion 20 when viewed from the axial direction of the outer space 25 is not limited to an approximately circular shape, and the shape thereof may be approximately oval or approximately elliptical, or may be approximately polygonal. Furthermore, although one tubular portion 20 is provided according to the present embodiment, a plurality of the tubular portions 20 may be provided. For example, two tubular portions 20 may be provided, and the two tubular portions 20 may be arranged in a so-called double helix shape.

According to the present embodiment, the plurality of through-holes 26 that penetrate through the tube wall 21 are formed in a region of the outer circumferential portion 23 of the tubular portion 20 that encloses the outer space 25, more specifically, a region that faces the inner side of the helical shape.

The through-holes 26 are provided so as to be distributed at a predetermined density in a region of the outer circumferential portion 23 that encloses the outer space 25. The through-holes 26 may be randomly arranged or may be arranged in a regular manner at predetermined intervals. The orientation at which the through-holes 26 penetrate through the tube wall 21 is not limited to an orientation along a normal line of the tube wall 21, and the orientation may be at a predetermined angle with respect to the normal line of the tube wall 21.

As one example according to the present embodiment, the through-holes 26 penetrate through the tube wall along the normal line of the tube wall 21, and are randomly arranged at a predetermined density.

A fixing section 30 is provided in the endoscope cleaning instrument 10 of the present embodiment. The fixing section 30 is a member for positioning at least the tubular portion 20 of the endoscope cleaning instrument 10, for example, inside the cleaning tank of the endoscope cleaning apparatus.

As one example according to the present embodiment, the fixing section 30 is provided on an outer circumferential portion of the proximal end 28 of the tubular portion 20, and has a configuration that engages with a support column 104 provided inside the cleaning tank to position the tubular portion 20.

More specifically, the fixing section 30 of the present embodiment includes two disc portions 31 and 32 that are provided so as to extend from the outer circumferential portion of the proximal end 28 of the tubular portion 20 to the circumference. The two disc portions 31 and 32 are arranged in an approximately parallel manner in a state in which the disc portions 31 and 32 are separated by a predetermined distance.

The fixing section 30 having the above configuration engages with the support column 104 provided inside the cleaning tank by, for example, sandwiching the support column 104 between the two disc portions 31 and 32, to thereby position the tubular portion 20 inside the cleaning tank.

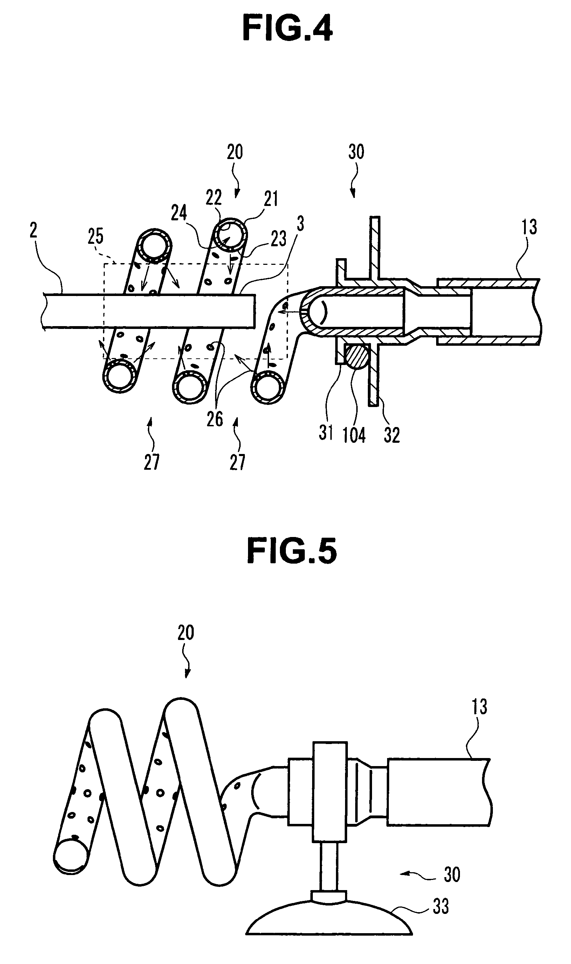

When the distal end portion 3 of the insertion portion 2 of an endoscope is to be cleaned using the endoscope cleaning instrument 10 of the present embodiment having the above described configuration, the connector 12 of the connection portion 11 is attached to the fluid supply port 102 of the fluid supply section 101. Next, as shown in Fig. 4, the distal end portion 3 is inserted into the outer space 25 enclosed by the helical tubular portion 20, and supply of a fluid from the fluid supply section 101 is started.

Thereupon, as indicated by the arrows in Fig. 4, the fluid is ejected from the plurality of through-holes 26 that are provided so as to connect the inner space 24 of the tubular portion 20 and the outer space 25 enclosed by the tubular portion 20. The fluid is ejected towards the inner side of the outer space 25 that accommodates the distal end portion 3.

In this case, because the plurality of through-holes 26 are randomly arranged in a region facing the outer space 25, of the tubular portion 20 that is constituted by a tubular member that has a circular cross-section which is formed in a helical shape, the fluid is ejected at various angles from at various positions from the outside of the outer space 25 towards the inside of the outer space 25.

Thus, according to the endoscope cleaning instrument 10 of the present embodiment, it is possible to clean off dirt that adheres to the distal end portion 3 by ejecting a fluid from various angles towards the distal end portion 3.

Since the endoscope cleaning instrument 10 of the present embodiment has a simple configuration in which a tubular member in which the plurality of through-holes 26 are provided is bendingly formed in a helical shape, the endoscope cleaning instrument 10 can be manufactured easily and at a low cost.

Further, according to the endoscope cleaning instrument 10 of the present embodiment, because the tubular portion 20 is a winding helical shape that has the clearance 27, the outer space 25 communicates with the outer side of the endoscope cleaning instrument 10 through the clearance 27. Consequently, according to the present embodiment it is easy to visually confirm that the distal end portion 3 is accommodated inside the outer space 25. It is also possible to easily check by visual observation the state of the process to clean off dirt from the distal end portion 3. Further, dirt that has been cleaned off from the distal end portion 3 can also be prevented from building up within the outer space 25. Thus, according to the endoscope cleaning instrument 10 of the present embodiment, the cleaning process of the distal end portion 3 can be performed more easily than heretofore.

Although according to the present embodiment, a configuration is adopted such that the fixing section 30 positions the tubular portion 20 with respect to a cleaning tank of an endoscope cleaning apparatus in which the support column 104 is provided by engaging with the support column 104, the form of the fixing section 30 is not limited thereto.

For example, as illustrated in Fig. 5, a configuration may be adopted such that the fixing section 30 includes a suction portion 33 that adheres to a wall surface of the cleaning tank. The suction portion 33 may be in the form of a so-called suction cup that adheres to a flat wall surface by means of a pressure difference, or may be in the form of a magnet that adheres to a wall surface by means of a magnetic force.

### (Second Embodiment)

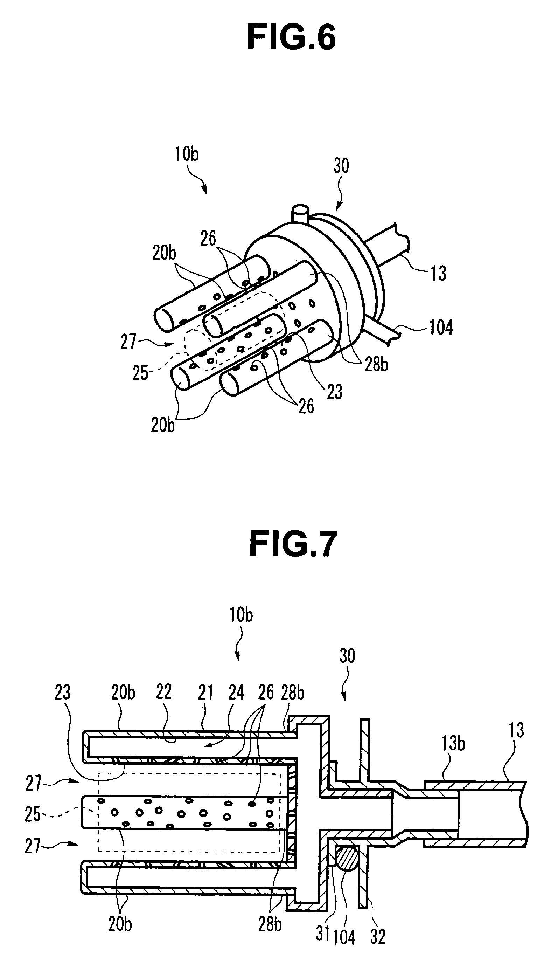

Hereunder, a second embodiment of the present invention is described referring to Fig. 6 and Fig. 7. In an endoscope cleaning instrument 10b of the present embodiment, the configuration of a tubular portion 20b is different to the first embodiment. Hereunder, only the differences with respect to the first embodiment are described, and components that are the same as in the first embodiment are denoted by like reference numerals, and a description of such components is omitted as appropriate.

The endoscope cleaning instrument 10b of the present embodiment includes at least three tubular portions 20b. The tubular portions 20b are arranged at predetermined intervals along the periphery of the outer space 25. Preferably, the tubular portions 20b are equally spaced around the circumference of the outer space 25, as viewed from the axial direction of the outer space 25.

As one example according to the present embodiment, four tubular portions 20b that are each constituted by an approximately linear tubular member are arranged along the periphery of the outer space 25. The four tubular portions 25 are equally spaced at angular intervals of approximately 90 degrees along the periphery of the outer space 25 as viewed from the axial direction of the outer space 25. According to the present embodiment, since the plurality of tubular portions 20b are arranged at predetermined intervals along the periphery of the outer space 25, clearances 27 are formed in which the outer space 25 that is enclosed by the plurality of tubular portions 20b and the external portion of the endoscope cleaning instrument 10b communicate.

The inner space 24 of each of the four tubular portions 25 connects to the distal end 13b of the connection tube portion 13 of the connection portion 11 at the respective proximal ends 28b of the four tubular portions. A plurality of through-holes 26 are randomly formed in a region facing the outer space 25 of the four tubular portions 20b. The plurality of through-holes 26 are formed at various angles with respect to the normal line of the tube wall 21.

According to the endoscope cleaning instrument 10b of the present embodiment that has the above described configuration, similarly to the first embodiment, a fluid supplied from the fluid supply section 101 is ejected at various angles from various positions towards the inner side of the outer space 25 from the outer side thereof.

Therefore, according to the endoscope cleaning instrument 10b of the present embodiment, dirt that adheres to the distal end portion 3 can be cleaned off by ejecting a fluid from various angles towards the distal end portion 3.

Further, since the endoscope cleaning instrument 10b of the present embodiment has a simple configuration in which the plurality of tubular portions 20b are arranged that are substantially linear-shaped tubular members in which the through-holes 26 are formed, the endoscope cleaning instrument 10b can be manufactured easily and at a low cost.

Furthermore, according to the endoscope cleaning instrument 10b of the present embodiment, because the plurality of tubular portions 20b are arranged so that the clearance 27 exists between each tubular portion 20b, the outer space 25 communicates with the outer side of the endoscope cleaning instrument 10b through the clearances 27. Consequently, according to the present embodiment it is possible to easily confirm visually that the distal end portion 3 is accommodated inside the outer space 25. It is also possible to easily check by visual observation a state of the process to clean off dirt from the distal end portion 3. Further, dirt that has been cleaned off from the distal end portion 3 can be prevented from building up within the outer space 25. Thus, according to the endoscope cleaning instrument 10b of the present embodiment, the cleaning process of the distal end portion 3 can be performed more easily than heretofore.

### (Third Embodiment)

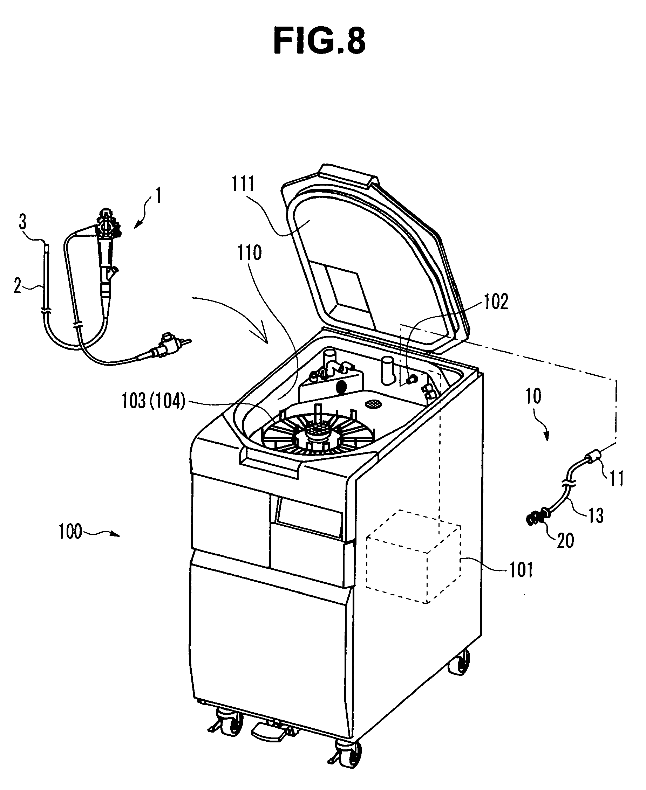

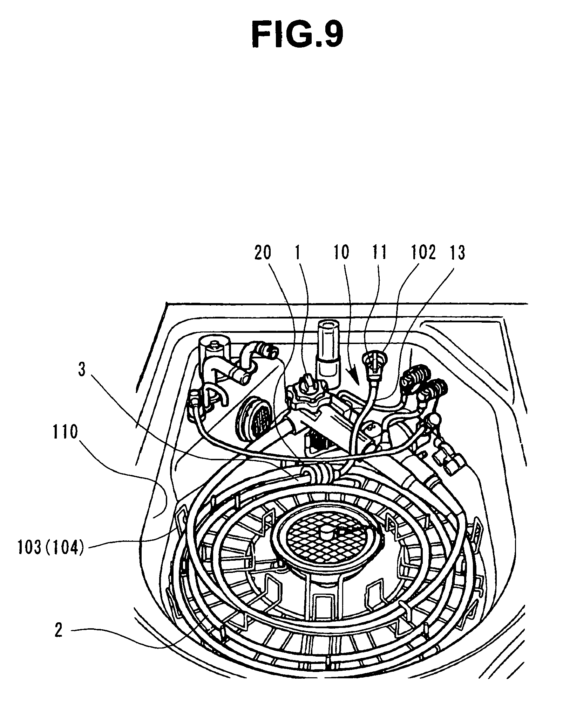

Hereunder, a third embodiment of the present invention is described referring to Fig. 8 and Fig. 9. Hereunder, only differences with respect to the first embodiment are described, and components that are the same as in the first embodiment are denoted by like reference numerals, and a description of such components is omitted as appropriate.

An endoscope cleaning apparatus 100 of the present embodiment includes the endoscope cleaning instrument 10, a cleaning tank 110 that can house an endoscope 1, and a liquid supply section 101 that supplies a liquid. The endoscope cleaning apparatus 100 is an apparatus that automatically performs a cleaning process using a fluid with respect to the endoscope 1 that is housed inside the cleaning tank 110.

The cleaning tank 110 is a tub-shaped member that has an opening that can be sealed shut by means of a lid portion 111. The fluid supply section 101 includes an electrically-driven pump and a storage tank that stores a fluid. The fluid supply section 101 supplies a fluid into the cleaning tank 110 via at least the fluid supply port 102. The fluid supply port 102 is arranged inside the cleaning tank 110, and is configured so that the connection portion 11 of the endoscope cleaning instrument 10 can be connected thereto.

A support net 103 is arranged inside the cleaning tank 110. As shown in Fig. 9, the support net 103 is a member for retaining the endoscope 1 at a predetermined orientation inside the cleaning tank 110. At least one part of the support net 103 has a shape that engages with the fixing section 30 of the endoscope cleaning instrument 10, and has a function as the support column 104 for positioning the tubular portion 20 of the endoscope cleaning instrument 10.

The remaining configuration of the endoscope cleaning apparatus 100 is the same as that of a known endoscope cleaning apparatus, and hence a detailed description thereof is omitted here.

When the endoscope 1 is to be cleaned using the endoscope cleaning apparatus 100 having the above described configuration, first the endoscope 1 is placed inside the cleaning tank 110. Next, the connection portion 11 of the endoscope cleaning instrument 10 is fitted to the fluid supply port 102. Subsequently, as shown in Fig. 9, the distal end portion 3 of the insertion portion 2 of the endoscope 1 is inserted into the outer space 25 that is enclosed by the tubular portion 20, and the automatic cleaning process by the endoscope cleaning apparatus 100 is started. The manner in which dirt of the distal end portion 3 of the insertion portion 2 of the endoscope 1 can be cleaned off during the cleaning process is the same as the manner described above in relation to the first embodiment.

Note that the process of cleaning the distal end portion 3 using the endoscope cleaning instrument 10 may be performed in a state in which fluid has been filled inside the cleaning tank 110 and the distal end portion 3 is in the fluid, or may be performed in a state in which fluid is not filled inside the cleaning tank 110.

According to the present embodiment, since the connection portion 11 includes a flexible connection tube portion 13, a position at which the tubular portion 20 is fixed inside the cleaning tank 110 can be freely changed. Therefore, even if the position of the distal end portion 3 inside the cleaning tank 110 is changed, the distal end portion 3 can be inserted into the outer space 25 that is enclosed by the tubular portion 20. It is possible that the position of the distal end portion 3 inside the cleaning tank 110 will be changed, for example, when cleaning an endoscope 1 that has an insertion portion 2 of a different length or in a case where the orientation of the endoscope 1 is different when the endoscope 1 is housed inside the cleaning tank 110.

Note that the present invention is not limited to the above-mentioned embodiments, but may be suitably changed without departing from the spirit or concept of the present invention readable from the appended claims and the entire specification, and an endoscope cleaning instrument and an endoscope cleaning apparatus with such changes are also included in the technical scope of the present invention.

### Industrial Applicability

As described above, the present invention is preferable for an endoscope cleaning instrument and an endoscope cleaning apparatus that clean an endoscope by using fluid.

The present application is filed claiming the priority of Japanese Patent Application No. 2009-212246 filed in Japan on September 14, 2009, and the above described disclosure is incorporated by reference in the present description, claims and drawings.

## Claims

1. An endoscope cleaning instrument that cleans a distal end portion of an endoscope by ejecting a fluid that is fed from a fluid supply section towards the distal end portion, comprising:
a connection portion that is attachable to and detachable from the fluid supply section;
at least one tubular portion that has a tube shape comprising an inner circumferential portion and an outer circumferential portion and that can introduce the fluid that is supplied from the fluid supply section via the connection portion into an inner space enclosed by the inner circumferential portion, and that is provided so as to enclose an outer space that is capable of accommodating the distal end portion by means of at least a part of the outer circumferential portion; and
a plurality of through-holes provided in the tubular portion so as to connect the inner space and the outer space.

2. The endoscope cleaning instrument according to claim 1, wherein the tubular portion has a helical-shaped portion formed in a helical shape so that the outer circumferential portion encloses the outer space.

3. The endoscope cleaning instrument according to claim 1, wherein at least three of the tubular portions are arranged along a periphery of the outer space.

4. The endoscope cleaning instrument according to claim 1, wherein the tubular portion is provided so as to have a clearance such that the outer space communicates with an external portion of the endoscope cleaning instrument.

5. The endoscope cleaning instrument according to claim 1, wherein the fluid supply section is an endoscope cleaning apparatus comprising a cleaning tank that can accommodate the endoscope, and comprises:
a fixing section that is attachable to and detachable from the cleaning tank and that performs positioning of the tubular portion inside the cleaning tank.

6. An endoscope cleaning apparatus comprising an endoscope cleaning instrument according to claim 1.
